# EUROPEAN PATENT APPLICATION

(11) **EP 1 811 022 A1**
(43) Date of publication of application: **25.07.2007**
(21) Application number: 05785206.3
(22) Date of filing: 26.09.2005
(51) Int. Cl.: C12N 15/09, C12N 1/21, C12N 9/78

(54) **ARYLACYLAMIDASE GENE AND METHOD OF USING THE SAME**

(30) Priority: 04.10.2004 JP 2004291916
(71) Applicant: Kaneka Corporation, Kita-ku Osaka-shi, Osaka 530-8288 (JP)
(72) Inventor: KIZAKI, Noriyuki, c/o Kaneka Corporation, Takasago-shi, Hyogo 676-8688 (JP); ITO, Noriyuki, c/o Kaneka Corporation, Takasago-shi, Hyogo 676-8688 (JP); YASOHARA, Yoshihiko, c/o Kaneka Corporation, Takasago-shi, Hyogo 676-8688 (JP); NAGASAWA, Toru, Gifu-shi, Gifu 5011174 (JP)
(74) Representative: Vossius & Partner
(86) International application number: PCT/JP2005/017575
(87) International publication number: WO 2006/038479

(57) **Abstract**

The present invention provides an efficient method of producing an arylacylamidase. In an aspect, the present invention provides (a) a DNA comprising the base sequence shown in the sequence listing under SEQ ID NO: 1, or (b) a DNA capable of hybridizing, under stringent conditions, with a DNA comprising the base sequence complementary to the base sequence shown in the sequence listing under SEQ ID N0:1 and coding for a polypeptide having arylacylamidase activity. In another aspect, the invention provides a polypeptide encoded by the DNA mentioned above and having arylacylamidase activity, a vector containing the DNA mentioned above, a transformant which is transformed using the DNA or the vector mentioned above, and a method for producing an arylacylamidase which comprises cultivating the transformant mentioned above and obtaining the arylacylamidase from the culture medium.

## Description

### TECHNICAL FIELD

The present invention relates to a DNA coding for an arylacylamidase and a method of arylacylamidase production utilizing the DNA.

### BACKGROUND ART

(CROSS-REFERENCE TO RELATED APPLICATION) All the contents, including the specification, claims, drawings and abstract, as disclosed in Japanese Patent Application 2004-291916 (filed October 4, 2004) are incorporated herein by reference.

Arylacylamidases are defined by the International Union of Biochemistry (Enzyme Nomenclature, 1978, Academic Press, New York) and are described as showing catalytic activity in the hydrolysis of anilides into aniline and fatty acid anions. Arylacylamidases are industrially useful enzymes utilizable in quantitating anilides such as acetaminophen (Patent Document 1) .

It is known that arylacylamidases occur in mammals, higher plants and microorganisms. However, mammal- and higher plant-derived such enzymes are not suited for commercial production for the reasons, among others, 1) that those animals or plants which are to serve as enzyme sources are not easily available, 2) that the procedure for isolation from animals or plants is complicated and 3) that it is difficult to produce them in host microorganisms using the recombinant DNA technology. On the contrary, microorganism-derived enzymes produce no such problems and are advantageous for commercial production in many cases.

The arylacylamidases reportedly isolated from microorganisms include those derived from Candida utilis (Non-Patent Document 1), from Bacillus sphaericus (Non-Patent Document 2), from Pseudomonas acidovorans (Non-Patent Document 3), from Pseudomonas fluorescens (Non-Patent Document 4), from Rhodococcus erythropolis (Patent Document 2), from Coryneform bacterium (Non-Patent Document 5), from Pseudomonas sp. (Non-Patent Document 6), and from Nocardia globerula (Patent Document 3; Non-Patent Document 7).

All the arylacylamidases disclosed in the above-cited documents can be isolated from the culture fluids prepared by cultivating the microorganisms serving as origins of the respective enzymes. However, the processes for producing those arylacylamidases are low in productivity per unit culture fluid quantity, hence can hardly be said to be of practical use. In addition, there is no information disclosed about the DNAs coding for these arylacylamidases and, therefore, it is impossible even to improve the productivity of these arylacylamidases by utilizing the recombinant DNA technology. Under such circumstances, a more efficient method of producing an arylacylamidase has been needed.
Patent Document 1: United States Patent No. 4,999,288
Patent Document 2: Japanese Kokai publication Sho-63-74484
Patent Document 3: Japanese Kokai Publication Hei-03-277281
Non-Patent Document 1: J. Gen. Microbiol., 59, 47-55 (1969)
Non-Patent Document 2: Appl. Microbiol., 26, 709-718 (1973)
Non-Patent Document 3: Eur. J. Biochem., 53, 357-369 (1975)
Non-Patent Document 4: Eur. J. Biochem., 132, 651-655 (1983)
Non-Patent Document 5: Journal of Pesticide Science, 18, 211-216 (1993)
Non-Patent Document 6: Sanop Misaengmul Hakhoechi, 26, 413-419 (1998)
Non-Patent Document 7: Eur. J. Biochem., 199, 17-24 (1991)

### SUMMARY OF THE INVENTION

It is an object of the invention to isolate a DNA coding for an arylacylamidase and utilize the same to provide an efficient method of producing the arylacylamidase.

The present inventors could isolate an arylacylamidase-encoding DNA from a microorganism having arylacylamidase activity and made the base sequence thereof clear. Further, they developed an arylacylamidase-producing transformant using that DNA and established an efficient method of producing the arylacylamidase with uses such transformant.

Thus, in an aspect, the present invention provides an arylacylamidase-encoding DNA isolated from a microorganism having arylacylamidase activity. In another aspect, the invention provides a polypeptide having arylacylamidase activity as encoded by that DNA. In a further aspect, the invention provides a vector containing that DNA. In a further aspect, the invention provides a transformant harboring that vector. In a still further aspect, the invention provides an arylacylamidase production method using that transformant.

### (EFFECTS OF THE INVENTION)

The present invention provides a practical method of producing arylacylamidases.

### DETAILED DESCRIPTION OF THE INVENTION

In the following, the present invention is described in detail. Unless otherwise specifically described, the procedures for DNA isolation, vector preparation, transformation and other gene manipulations described herein can be carried out by the methods described in such monographs as Molecular Cloning, 2nd Edition (Cold Spring Harbor Laboratory Press, 1989). Unless otherwise specified, "%" as used herein means "% (w/v)".

### 1. DNA

The DNA of the invention is a DNA coding for an arylacylamidase which may contain any arbitrary untranslated region provided that the DNA can express the arylacylamidase in host cells harboring the DNA as introduced therein by the method described later herein. Such DNA can be isolated from a microorganism having arylacylamidase activity. The microorganism to serve as an origin of the DNA of the invention is not particularly restricted but, for example, the DNA in question can be isolated from the Nocardia globerula NBRC 13510 strain. This microorganism can be obtained from the NITE Biological Resource Center (NBRC), Department of Biotechnology, National Institute of Technology and Evaluation (2-5-8 Kazusa Kamatari, Kisarazu City, Chiba Prefecture, 292-0818 Japan).

### 2. DNA isolation

The DNA of the invention can be isolated from a microorganism serving as the origin of that DNA, for example, by the method given below.

First, the microorganism to serve as the origin of the DNA of the invention is cultivated using an appropriate medium. As for the medium for cultivating the microorganism, an ordinary liquid nutrient medium containing carbon sources, nitrogen sources, inorganic salts, organic nutrients and so forth can be used provided that the microorganism can grow therein.

Second, the arylacylamidase is isolated from the microorganism by using an appropriate combination of generally known protein purification techniques. For example, cells are collected from the culture fluid containing the microorganism by centrifugation or filtration, and the cells collected are disrupted by physical means using a sonicator or glass beads, for instance, and the cell debris is removed by centrifugation to give a cell-free extract, which is subjected to fractional precipitation, ion exchange chromatography, hydrophobic chromatography, gel filtration chromatography, reversed phase chromatography and/or ultrafiltration, for instance, whereupon the arylacylamidase can be isolated.

The activity of the arylacylamidase can be measured, for example, by adding 10 µl of a test solution to 990 µl of 100 mM glycine buffer (pH 9.5) containing 10 mM of 2-methylacetanilide (reaction substrate), allowing the reaction to proceed at 35°C for 20 minutes, adding 0.2 ml of 1 M hydrochloric acid to terminate the reaction and assaying 2-methylaniline formed in the reaction mixture by HPLC (column: M&S Pack C18 (⌀ 4. 6 x 150 mm) ; product of M&S Instruments Inc.), mobile phase: acetonitrile/1% aqueous potassium dihydrogen phosphate solution = 3/7, detection: 240 nm, flow rate: 1 ml/minute).

Third, the amino acid sequence of the arylacylamidase isolated is partially determined. For example, the arylacylamidase isolated is subjected to appropriate endopeptidase digestion, and the peptide fragments formed are separated by reversed phase HPLC. Among them, an appropriate fragment is analyzed using a type ABI 492 protein sequencer (product of Applied Biosystems) . In this manner, the amino acid sequence of the arylacylamidase can be partially determined.

Fourth, the arylacylamidase-encoding DNA is partially amplified by PCR (polymerase chain reaction), and the base sequence of the portion amplified is determined. This step can be carried out, for example, in the following manner. First, based on the information about the amino acid sequence as obtained in the manner described above, PCR primers for partial amplification of the arylacylamidase-encoding DNA are synthesized. Then, the chromosomal DNA of the microorganism to serve as the origin of the above DNA is prepared by a conventional method of DNA isolation, for example the method of Visser et al. (Appl. Microbiol. Biotechnol., 53, 415 (2000)). Using this chromosomal DNA as the template, the PCR is carried out using the above-mentioned PCR primers to thereby partially amplify the arylacylamidase-encoding DNA. Further, the base sequence of the amplified DNA is determined using a type ABI 373A DNA sequencer (product of Applied Biosystems), for instance.

Fifth, based on the partial base sequence of the arylacylamidase-encoding DNA as revealed in the above manner, the whole base sequence thereof is determined, for example, by the i-PCR method (Nucl. Acids Res., 16, 8186 (1988)).

As examples of the DNA of the invention which can be obtained in the above manner, there may be mentioned DNAs comprising the base sequence shown in the sequence listing under SEQ ID N0: 1 . The DNA of the invention further includes any DNA capable of hybridizing, under stringent conditions, with a DNA comprising the base sequence complementary to the base sequence shown in the sequence listing under SEQ ID NO:1 and coding for a polypeptide having arylacylamidase activity.

The DNA capable of hybridizing, under stringent conditions, with a DNA comprising the base sequence complementary to the base sequence shown in the sequence listing under SEQ ID NO:1 refers to a DNA with which a DNA comprising the DNA complementary to the base sequence shown in the sequence listing under SEQ ID N0: 1 specifically forms a hybrid when the colony hybridization method, plaque hybridization method or southern hybridization method, for instance, is carried out.

The stringent conditions so referred to herein refer, for example, to conditions such that the hybridization is carried out in an aqueous solution having a composition comprising 75 mM trisodium citrate, 750 mM sodium chloride, 0.5% sodium dodecyl sulfate, 0.1% bovine serum albumin, 0.1% polyvinylpyrrolidone and 0.1% Ficoll 400 (product of Amersham Biosciences) at 65°C, followed by washing at 60°C using an aqueous solution having a composition comprising 15 mM trisodium citrate, 150 mM sodium chloride and 0.1% sodium dodecyl sulfate. Preferably, the conditions are such that the hybridization carried out under the conditions mentioned above is followed by washing at 65°C with an aqueous solution having a composition comprising 15 mM trisodium citrate, 150 mM sodium chloride and 0.1% sodium dodecyl sulfate and, more preferably, such that the hybridization carried out under the conditions mentioned above is followed by washing at 65°C with an aqueous solution having a composition comprising 1.5 mM trisodium citrate, 15 mM sodium chloride and 0.1% sodium dodecyl sulfate.

### 3. Polypeptide

The polypeptide of the invention is a polypeptide encoded by the DNA of the invention and having arylacylamidase activity. As an example of the polypeptide of the invention, there may be mentioned a polypeptide comprising the amino acid sequence shown in the sequence listing under SEQ ID N0:2 and encoded by the base sequence shown under SEQ ID N0: 1 in the sequence listing. However, the polypeptide of the invention is not limited thereto but includes any polypeptide that is encoded by a DNA capable of hybridizing, under stringent conditions, with a DNA having the base sequence complementary to the base sequence shown in the sequence listing under SEQ ID NO: 1 and has arylacylamidase activity.

Such polypeptide is obtained, for example, by joining the above-mentioned DNA capable of hybridizing, under stringent conditions, with a DNA having the base sequence complementary to the base sequence shown in the sequence listing under SEQ OD NO:1 to an appropriate vector and introducing the ligation product into appropriate host cells for expression of the polypeptide. Such a desired polypeptide can also be obtained by causing amino acid substitution, insertion, deletion or addition to occur in the polypeptide having the amino acid sequence shown in the sequence listing under SEQ ID NO:2 according to such a known method as described in Current Protocols in Molecular Biology (John Wiley and Sons, Inc., 1989) or elsewhere. Unless the arylacylamidase activity is lost, the number of amino acids to be substituted, inserted, deleted or added is not restricted but is, for example, not greater than 20 amino acids, preferably not greater than 5, more preferably 2 or 1.

### 4. Vector

The vector of the invention is a vector containing the DNA of the invention. The vector for introducing the DNA of the invention into a host microorganism for polypeptide expression in the host microorganism resulting from the DNA introduction is not particularly restricted provided that the gene encoded in that DNA can be expressed in the appropriate host microorganism. Such vector includes, among others, plasmid vectors, phage vectors and cosmid vectors and, further, shuttle vectors capable of gene exchange with some other host strain can also be used. Such vectors generally contain such a regulatory factor as the lacUV5 promoter, trp promoter, trc promoter, tac promoter, lpp promoter, tufB promoter, recA promoter or pL promoter and can be properly used as expression vectors containing an expression unit operably linked to the DNA of the invention. For example, pUCNT (WO 94/03613) can suitably be used.

The term "regulatory factor" as used herein refers to a base sequence comprising a functional promoter and an arbitrary related transcription element (e.g. enhancer, CCAAT box, TATA box, SPI site).

The phrase "operably linked" as used herein means that various regulatory elements such as promoters, enhancers and so forth, which control gene expression, are linked to the gene in a state such that they are operable in host cells. It is well known to those skilled in the art that the type and kind of regulatory factor vary depending on the host species.

As an example of the vector of the invention, there may be mentioned pNTNG, which is described later herein. pNTNG is obtained by inserting the DNA defined under SEQ ID NO:1 into the above-mentioned expression vector pUCNT.

### 5. Transformant

The transformant of the invention is obtained by introducing the vector of the invention into host cells. As the host cells into which the vector of the invention is to be introduced, there may be mentioned bacteria, yeasts, filamentous fungi, plant cells and animal cells, among others. Escherichia coli is particularly preferred, however. The vector of the invention can be introduced into host cells by a conventional method. When Escherichia coli cells are used as the host cells, commercially available competent E. coli HB101 cells (product of Takara Bio Inc.), for instance, can be used to introduce the vector in question into the host cells.

As the transformant of the invention, there may be mentioned E. coli HB101(pNTNG) FERM BP-10416, which is to be described later herein. This transformant has been deposited with the National Institute of Advanced Industrial Science and Technology International Patent Organism Depositary (Chuo Dai-6, 1-1-1 Higashi, Tsukuba City, Ibaraki Prefecture, 305-8566 Japan) as of September 15, 2005 under the accession number given above (the strain nationally deposited originally on October 23, 2003 has been transferred to the international depository under the Budapest Treaty).

An arylacylamidase can be produced in good yields by cultivating the transformant of the invention. The cultivation of the transformant of the invention can be conducted using any ordinary liquid nutrient medium containing carbon sources, nitrogen sources, inorganic slats, organic nutrients and so forth provided that the transformant can grow. The arylacylamidase yield per unit culture fluid volume can be improved by adding any of various inducers to the medium, if necessary. The arylacylamidase accumulated in the culture fluid resulting from cultivation of the transformant of the invention can be purified or semi-purified for use by using one or more conventional protein purification techniques, although the culture fluid as such can also be used as an arylacylamidase-containing product.

### BEST MODE FOR CARRYING OUT THE INVENTION

The following examples illustrate the present invention in detail. These examples are, however, by no means limitative of the scope of the invention.

### (Example 1) Determination of base sequence of arylacylamidase-encoding DNA

### (PCR primer preparation)

Based on the information about a partial amino acid sequence of the Nocardia globerula NBRC 13510 strain-derived arylacylamidase (HNG) as disclosed in Eur. J. Biochem., 199, 17-24 (1991), primer 1: 5'-atggaygtngcngartaygc-3' (SEQ ID N0:3) and primer 2: 5'-acytcrcangcrctnacytg-3' (SEQ ID N0:4) were synthesized for amplifying a part of the arylacylamidase-encoding DNA by PCR.

### (Amplification of arylacylamidase-encoding DNA)

According to the method of Visser et al. (Appl. Microbiol. Biotechnol., 53, 415 (2000)), chromosomal DNA was extracted from cells of the Nocardia globerula NBRC 13510 strain cultivated by the method disclosed in Eur. J. Biochem., 199, 17-24 (1991) . Then, PCR was carried out using the DNA primers 1 and 2 prepared as described above and the chromosomal DNA obtained as the template, whereupon a DNA fragment having a size of about 80 bp, which was thought to be a part of the desired gene, was amplified. The PCR was carried out using TaKaRa Ex Taq (product of Takara Bio Inc.) as a DNA polymerase under the reaction conditions recommended in the manual attached to the polymerase. This DNA fragment was cloned into the plasmid pT7Blue T-Vector (product of Novagen), and the base sequence thereof was determined using ABI PRISM Dye Terminator Cycle Sequencing Ready Reaction Kit (product of Perkin Elmer) and ABI 373A DNA Sequencer (product of Perkin Elmer) . The base sequence determined is shown in the sequence listing under SEQ ID NO:5. (Determination of full-length sequence of HNG gene by i-PCR technique)

The chromosomal DNA of the Nocardia globerula NBRC 13510 strain was completely digested using the restriction enzyme ApaLI, EcoRI or SphI, and the digestion products obtained were each intramolecularly cyclized using T4 DNA ligase (product of Takara Bio Inc.). Using these products as templates and based on the information about the partial base sequence of the arylacylamidase-encoding DNA as above revealed, the whole base sequence of the arylacylamidase-encoding DNA was determined by carrying out the i-PCR method (Nucl. Acids Res., 16, 8186 (1988)). The i-PCR was carried out using TaKaRa Ex Taq (product of Takara Bio Inc.) as a DNA polymerase under the-reaction conditions recommended in the manual attached thereto. The base sequence determination was performed in the same manner as described above. The base sequence revealed is shown in the sequence listing under SEQ ID NO:1. The amino acid sequence encoded by that base sequence is shown in the sequence listing under SEQ ID NO:2.

### (Example 2) Expression vector construction

PCR was carried out using primer 3: 5'-gtgcatatggatgtcgccgaatacgc-3' (SEQ ID NO: 6) and primer 4: 5'-gacggatccttactaccggcccacgtgcacgg-3' (SEQ ID NO: 7) and the chromosomal DNA of the Nocardia globerula NBRC 13510 strain as obtained in Example 1 as the template. As a result, a double-stranded DNA resulting from addition of an NdeI recognition site to the initiation codon region and addition of a BamHI recognition site just behind the termination codon of the DNA having the base sequence shown in the sequence listing under SEQ ID NO:1 was obtained. The PCR was carried out using TaKaRa La Taq (product of Takara Bio Inc.) as a DNA polymerase under the reaction conditions recommended in the manual attached thereto. This DNA was digested with NdeI and BamHI, followed by insertion between the NdeI recognition site and BamHI recognition site downstream from the lac promoter of the plasmid pUCNT (WO 94/03613), whereby a recombinant vector, pNTNG, was thus constructed.

### (Example 3) Transformant production

Competent E. coli HB101 cells (product of Takara Bio Inc.) were transformed with the recombinant vector pNTNG constructed in Example 2 to give E. coli HB101 (pNTNG). This transformant has been deposited with the National Institute of Advanced Industrial Science and Technology International Patent Organism Depositary as of September 15, 2005 under the accession number FERM BP-10416 (the strain nationally deposited originally on October 23, 2003 has been transferred to the international depository under the Budapest Treaty).

### (Example 4) Arylacylamidase production by transformant

2 x YT medium (1.6% tryptone, 1.0% yeast extract, 0.5% NaCl, pH 7.0) containing 200 µg/ml of ampicillin was sowed with the transformant E. coli HB101(pNTNG) obtained in Example 3, and the transformant was cultivated at 37°C for 24 hours. Cells were harvested from 1 ml of this culture fluid by centrifugation and suspended in 1 ml of 100 mM phosphate buffer (pH 6.5) . The cells were disrupted using a model UH-50 ultrasonic homogenizer (product of SMT Co.) and the cell debris was removed by centrifugation to give a cell-free extract. The arylacylamidase activity and protein concentration of this cell-free extract were measured. The activity of each milligram of the protein was 0.43 U. On the other hand, the corresponding activity of the cell-free extract of the transformant E. coli HB101(pUCNT) resulting from introduction of the vector plasmid free of the arylacylamidase-encoding DNA as measured in the same manner was lower than 0.01 U per milligram of protein.

The arylacylamidase activity was measured by adding 10 µl of the test solution to 990 µl of 100 mM glycine buffer (pH 9.5) containing 10 mM2-methylacetanilide (reaction substrate), allowing the reaction to proceed at 35°C for 20 minutes, then adding 0.2 ml of 1 M hydrochloric acid to terminate the reaction, and assaying 2-methylaniline formed in the reaction mixture by HPLC (column: M&S Pack C18 (⌀ 4.6 x 150 mm; product of M&S Instruments Inc.), mobile phase: acetonitrile/1% aqueous potassium dihydrogen phosphate solution = 3/7, detection: 240 nm, flow rate: 1 ml/minute). The activity forming 1 micromole of 2-methylaniline per minute under those activity measurement conditions was defined as 1 U. The protein concentration was determined using a protein assay kit (product of BIO-RAD).

### INDUSTRIAL APPLICABILITY

The present invention provides a practical method of producing arylacylamidases.

## Claims

1. A DNA consisting in the following (a) or (b):
(a) a DNA comprising the base sequence shown in the sequence listing under SEQ ID N0:1,
(b) a DNA capable of hybridizing, under stringent conditions, with a DNA comprising the base sequence complementary to the base sequence shown in the sequence listing under SEQ ID NO:1 and coding for a polypeptide having arylacylamidase activity.

2. A DNA which codes for a polypeptide comprising the amino acid sequence shown in the sequence listing under SEQ ID N0:2.

3. A polypeptide encoded by the DNA according to Claim 1 and having arylacylamidase activity.

4. A polypeptide comprising the amino acid sequence shown in the sequence listing under SEQ ID NO:2.

5. A vector containing the DNA according to Claim 1 or 2.

6. The vector according to Claim 5
which is a plasmid pNTNG.

7. A transformant which is obtained by transforming a host cell using the DNA according to Claim 1 or 2, or the vector according to Claim 5 or 6.

8. The transformant according to Claim 7
wherein the host cell is Escherichia coli.

9. The transformant according to Claim 8
which is E. coli HB101(pNTNG) FERM BP-10416.

10. A method for producing an arylacylamidase
which comprises cultivating the transformant according to any one of Claims 7 to 9 in a nutrient medium, and obtaining the arylacylamidase from the culture medium obtained.
